# EUROPÄISCHE PATENTSCHRIFT

(11) **EP 2 227 330 B1**
(45) Veröffentlichungstag und Bekanntmachung des Hinweises auf die Patenterteilung: **30.07.2014**
(21) Anmeldenummer: 08864313.5
(22) Anmeldetag: 22.12.2008
(51) Int. Cl.: B01L 7/00, B01L 3/00, C12Q 1/68

(54) **MOBILES SCHNELLTESTSYSTEM FÜR DIE NUKLEINSÄUREANALYTIK**
MOBILE RAPID TEST SYSTEM FOR NUCLEIC ACID ANALYSIS
SYSTÈME DE TEST RAPIDE MOBILE POUR ANALYSE D'ACIDE NUCLÉIQUE

(30) Priorität: 22.12.2007 DE 102007062441
(43) Veröffentlichungstag der Anmeldung: 15.09.2010
(73) Patentinhaber: AJ Innuscreen GmbH, 13125 Berlin (DE)
(72) Erfinder: HILLEBRAND, Timo, 15366 Hoppegarten (DE); KNIPPSCHILD, Claus, 07745 Jena (DE); JASCHINSKY, Benjamin, 06114 Halle (DE); GRASER, Elmara, 13086 Berlin (DE)
(74) Vertreter: Wehlan, Helmut
(86) Internationale Anmeldenummer: PCT/EP2008/068197
(87) Internationale Veröffentlichungsnummer: WO 2009/080817

(56) Entgegenhaltungen:
- WO-A-2004/065010
- WO-A-2004/099438
- WO-A-2007/092713
- WO-A-2007/116298
- WO-A-2009/000764
- WO-A2-2007/078850
- US-A1- 2005 227 275
- US-A1- 2006 252 064
- US-A1- 2007 054 296
- US-A1- 2007 166 204
- US-B1- 6 602 473
- GLYNOU K ET AL: "OLIGONUCLEOTIDE-FUNCTIONALIZED GOLD NANOPARTICLES AS PROBES IN A DRY-REAGENT STRIP BIOSENSOR FOR DNA ANALYSIS BY HYBRIDIZATION" ANALYTICAL CHEMISTRY, AMERICAN CHEMICAL SOCIETY. COLUMBUS, US, Bd. 75, Nr. 16, 15. August 2003 (2003-08-15), Seiten 4155-4160, XP001175793 ISSN: 0003-2700
- WITTWER C T ET AL: "Real-time multiplex PCR assays" METHODS : A COMPANION TO METHODS IN ENZYMOLOGY, ACADEMIC PRESS INC., NEW YORK, NY, US, Bd. 25, Nr. 4, 1. Dezember 2001 (2001-12-01), Seiten 430-442, XP002265718 ISSN: 1046-2023

## Beschreibung

Gegenstand der Erfindung ist ein mobiles Gerätesystem für die Gendiagnostik.

### Stand der Technik

Die Untersuchung diagnostisch relevanter biologischer Proben wie Serum, Plasma, Blut, Tupferproben oder Organabriebe zum Nachweis infektiöser Erreger hat in den letzten Jahren enorm an Bedeutung gewonnen. Virusinfektionen wie HIV, HCV oder HBV sind weltweit auf dem Vormarsch. Darüber hinaus sind auch bakterielle Infektionen wieder auf dem Vormarsch, u.a. auch als Ergebnis beginnender klimatischer Veränderungen. Das Auftreten neuer, tödlich verlaufender Infektionskrankheiten mit einem extrem hohen Infektiösitätspotential (SARS, Vogelgrippe) zeigt immer deutlicher, dass eine schnell und vor Ort durchführbare Diagnostik entscheidend für die Verhinderung von Epidemien sein wird. Darüber hinaus spielen einfach handhabbare und relativ preiswerte diagnostische Systeme, vor allem für Entwicklungsländer, ebenfalls eine bedeutende Rolle bei der Bekämpfung der Ausbreitung von Infektionskrankheiten. Die derzeitig vor allem für die Detektion viraler Infektionskrankheiten (HIV, HCV, HBV) eingesetzten Tests basieren mehrheitlich auf der Durchführung von REAL-TIME-PCR's. Diese Tests sind an extrem teure gerätetechnische Voraussetzungen gebunden sowie auch an teure Reagenzien. Die Durchführung dieser Methoden kann nur durch geschultes Fachpersonal in Speziallaboratorien erfolgen. Eine Vor-Ort-Diagnostik ist nicht möglich. Neue Generationen von integrativen Systemlösungen (Kombination von Nukleinsäureisolierung, Amplifikation und Chipdetektion) für eine mobile Vor-Ort-Diagnostik befinden sich in der Entwicklung, nicht aber in einem Stadium der erfolgreichen Vermarktung. Darüber hinaus fokussieren diese Systeme auch oftmals auf den Bereich der militärischen Diagnostik und sind auch in Analogie zu den "klassischen REAL-TIME-PCR"-Verfahren sehr teuer, sowohl geräte- als auch reagenzienseitig.

Die so-genannte Rapid-PCR-Technologie erlaubt die Durchführung von Amplifikationsreaktionen innerhalb von nur wenigen Minuten und ist damit deutlich schneller als Standard-PCR-Verfahren. Für die miniaturisierte Gerätelösung wird die Erfindung durch eine neuartige Reaktionskavität (Consumable) gelöst. Im Bereich der PCR-Consumables sind verschiedene Methoden zu deren Herstellung bekannt. Am meisten verbreitet ist das Herstellungsverfahren durch Spritzguss. Die so hergestellten Consumables sind in einer Vielzahl von Formen und Anordnungen kommerziell verfügbar. Diese Gefäße verfügen jedoch alle über eine sehr dicke Wandstärke (ca.0,2 mm-0,35 mm), welche einem guten Wärmetransfer, von dem beheizten Probenblock in die Probe, als ein sehr großer Widerstand entgegen steht. Werden diese Gefäße verwendungsgemäß in einem Thermocycler benutzt, heizt sich der in den Geräten befindliche Probenblock mit einer Geschwindigkeit von bis zu 5°C/s auf. Die resultierende Geschwindigkeit in der Probe wird jedoch durch die Gefäße erheblich vermindert, so dass die Probe (im Gefäß befindlich) nur mit ca. 1,5-2°C/s aufgeheizt wird. Selbst als "dünnwandig" (minimale Wandstärke von ca. 0,19 mm; Eppendorf Produktkatalog 07-08, S.200) angebotenen Gefäße vermögen es nicht, diesen Zustand erheblich zu verbessern. Die technischen Voraussetzungen (heizen und kühlen) für eine schnelle Abarbeitung der Temperaturen in der PCR sind technisch realisiert, werden jedoch durch das verwendete Consumable in seiner Effektivität stark beeinträchtigt.

Übliche in der PCR verwendete Probenvolumina liegen im Bereich von 5-50 µl. Die durch Spritzguss hergestellten Gefäße besitzen am Boden ihre maximale Wandstärke, so dass eine Temperierung und die Durchführung einer PCR Reaktion von geringen Volumina 1-10 µl (welche sich am Boden der Gefäße sammeln) nur sehr ungenau und langsam durchzuführen ist.

Die verwendeten metallischen Probenblöcke (Aluminium, Silber) in Thermocyclern mit sehr großen Wärmekapazität sorgen ferner dafür, dass schnelle Temperaturwechsel in der Probe auch bei der Verwendung von starken Heizelementen unmöglich zu realisieren sind. Die Schnelligkeit von bestehenden Systemen am Markt, die auf der Basis von metallischen Probenblöcken und Spritzguss Consumables arbeiten, stößt damit an objektive Grenzen. Daraus folgt, dass aufgrund ihrer enormen thermischen Ineffizienz miniaturisierte, mobile Varianten dieser Thermocycler die elektrische Leitungsfähigkeit zugänglicher Batterietechnologie in unzumutbarer Weise überschreitet.

Andere stationäre Systeme setzen auf eine fortgeschrittene Technologie, um die thermische Effizienz zu erhöhen und eine Rapid-PCR möglich zu machen.

Als das bekannteste kommerziell verfügbare schnelle PCR System sei hier der LightCycler von Roche Molecular Biochemicals genannt (cat. No. 1909339 und cat. No. 2011468). Dieses System beruht auf der Verwendung von sehr dünnen Glaskapillaren als PCR-Consumable und führt eine Temperierung mittels heißer Luft durch, welche die Kapillare umströmt. Diese Kapillaren können ein Volumen von 10-20 µl aufnehmen und zeichnen sich durch ihre große Oberfläche aus, die einen guten Wärmetransfer ermöglicht. Diese große Glasoberfläche adsorbiert jedoch Komponenten von Standard PCR-Ansätzen und lässt somit die Reaktion inaktiver werden. Um diesen Effekt zu kompensieren, müssen z.B. verschiedene Carrier Moleküle usw. eingesetzt werden (EP 1133359). Als ein weiterer Nachteil stellt sich hierbei ebenfalls die Handhabung der sehr dünnen Kapillare und deren Preis heraus. Eine Miniaturisierung und an eine einfache Handhabung der Gefäße ist jedoch weiterhin unmöglich.

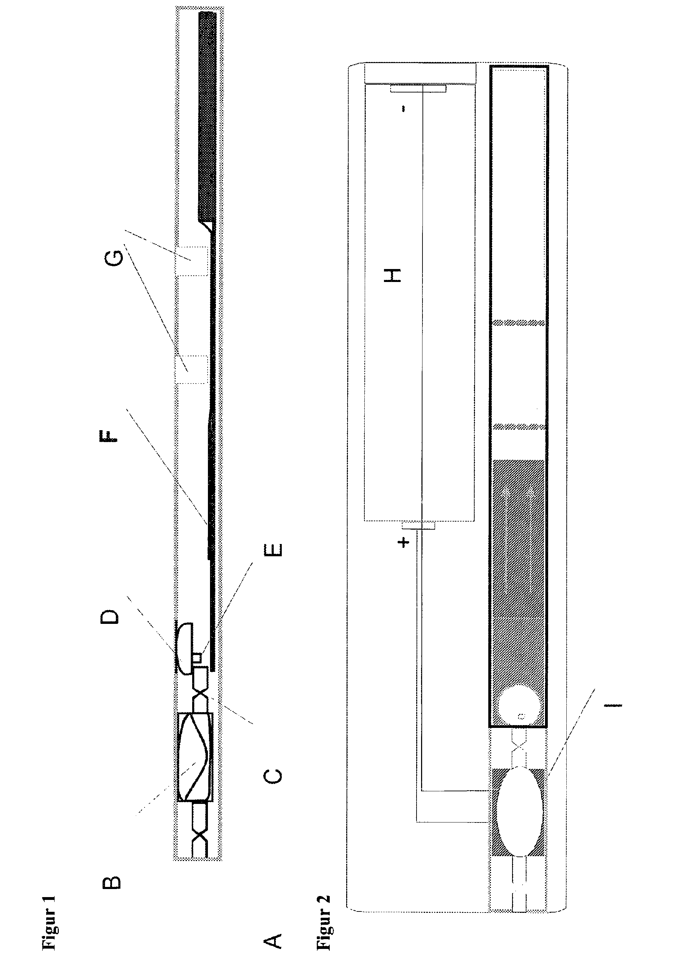

Eine Technologie, die nicht auf Standard-Consumables aufbaut und für den mobilen Einsatz genutzt werden kann, wird in der Offenlegungsschrift US2005/0227275 A1 erläutert. Hierin wird in die Wand der PCR-Reaktionskammer produktionstechnisch ein Metallgewebe eingebracht. Dieses sorgt für eine unmittelbare Wärmeübertragung zur Probe. Obwohl darauf hingewiesen wird, dass die Wand dünn sein soll, wird nicht ausgeführt, wie dünn. Gleichzeitig wird in keiner Weise auf die Kühlung der Probe eingegangen. Damit erklärt diese Schrift zwar, dass eine Rapid-PCR durchgeführt werden soll, jedoch lässt sie sich über die technische Umsetzung nur unzureichend aus. Unter diesen Punkt fällt auch die Tatsache, dass das dort beschriebene Consumable den Vorgaben, ein kostengünstiges Verbrauchsmaterial zu sein, nicht gerecht wird. Die Einbringung eines definierten Heizgewebes und einer Vorrichtung zur Temperaturkontrolle stehen dieser Vorgabe im Wege. Erfindungsgemäßer Gegenstand der Offenlegungsschrift US2005/0227275 A1 ist weiterhin die Durchführung einer PCR Reaktion und ein Nachweis von Amplifikationsprodukten mittels eines Lateral Flow Streifens. Es wird eine PCR mit markierten Primern und Nukleotiden beschrieben (Anspruch 1, Abb. 1, 2, 3, 4, 6). Nachteilig an diesem Verfahren ist aber, dass das PCR-Produkt nicht mit einer markierten Sonde hybridisiert wird. Der alleinige Nachweis eines Amplifikationsproduktes auf diesem Wege ist aber diagnostisch sehr unsicher, da die geforderte 100%ige Spezifität des Amplifikationsproduktes nicht gewährleistet ist. Dazu bedarf es einer spezifischen Hybridisierungsreaktion. Darüber hinaus besteht die latente Gefahr, dass falschpositive Resultate durch Mispriming und Primer-Dimere auftreten.

Die Verwendung eines "Chip" ähnlichen Designs zur Unterbringung einer PCR-Kammer stellt aufgrund der vielfachen Zitierung in der Patentliteratur keine erfinderische Höhe dar. Vielmehr stellt die Gestaltung des Heiz-/Kühlmechanismus, des Gesamtwärmeübergangs, die Zuführung der flüssigen/festen biochemischen Reaktionsbestandteile und die Umsetzung der Prozessparametersteuerung eine Neuerung dar. So geht z.B. die Druckschrift WO 2007/092713 A2 auf ein chipförmiges Consumable-Design ein, dass neben Zellsortierung und immunologischem Proteinnachweis auch eine PCR-Kammer umfassen kann. Mittels eines Lateral-Flow-Teststreifen-Verfahren werden unterschiedliche Zelltypen (Vorläufer von Krebszellen und Krebszellen) mit Hilfe entsprechender Antikörper separiert und diskriminiert. Das Lateral-Flow-Verfahren dient aber nicht dem Nachweis von Amplifikationsereignissen. Der Nachweis einer RNA-Expression aus RNA, welcher zuvor in einem sog. Labon-a Chip-System erfolgte, wird abgekoppelt von diesem Lab-on-a-Chip und erfolgt beschriebener weise am "Labortisch mit an sich bekannten Methoden". Beschrieben wird, dass alternativ dazu ein Amplifikations-Reaktor und ein Detektor Bestandteil des Lab-on-a-Chip-Systems sein kann. Auf die Ausgestaltung der PCR-Kammer im Sinne der oben genannten Eigenschaften wird in dieser Druckschrift nicht eingegangen. Auch findet sich keine Erwähnung einer PCR-Reaktion innerhalb der Patentansprüche. Nichts desto trotz lässt sich hier eine mobile Variante des Gerätesystems (mit Consumable) diskutieren. Für einen netzunabhängigen Betrieb des Gerätes muss die Leistungsaufnahme prozessoptimiert und so gering wie möglich sein. Durch die Verwendung einer Vielzahl von thermoelektrischen Modulen ("Hydrogel-Ice Valves") und fluidischer Pumpen erkennt der Fachmann, dass dieses Gerät nicht für den mobilen Batteriebetrieb geeignet sein kann.

Die Autoren der zuvor beschriebenen Druckschrift gehen in einer Publikation genauer auf die Thematik der Chipherstellung und der PCR-Kammer innerhalb dieses bzw. eines ähnlichen Chip ein (Chen, Z. et al.; Ann.N.Y. Acad. Sci. (March 2007)1098; 429-436). Es wird ein System vorgestellt, welches eine PCR Kammer und einen Lateral Flow Test-Streifen umfasst. Gemessen an den Vorgaben - schnell (Rapid-PCR), mobil (Batteriebetrieb), kostengünstig (Consumable) und handhabbar - muss diese Erfindung wie folgt bewertet werden. Dem Experten ist bekannt, dass eine Rapid-PCR durch die Gesamtdauer der Reaktion (mit 30 Zyklen in weniger als 30 Minuten) und durch die erreichten Temperaturwechsel in der Probe (> 4 K/Sekunde) definiert wird. Ohne die Angabe von Heiz- bzw. Kühlraten, einer Zykluszeit von mindestens einer Minute (Summe der Haltezeiten ohne Dauer des Temperaturwechsels) sowie der Verwendung einer frästechnisch gefertigten PCR-Kammer aus Polycarbonat (Wanddicken von mindestens 100 µm) wird dem Fachmann erklärlich, dass dieses Gerätesystem nicht Rapid-PCR tauglich ist. In dieser Publikation wird in keiner Weise ein mobiles System benannt. Ebenso wie in der davor beschriebenen Patentschrift sollen hier geräteseitig mehr als 10 thermoelektrische Module, dazu zwei Mehrwege-Stellventile, eine Flüssigkeitspumpe, eine Vakuumpumpe sowie ein Laserscanner untergebracht sein. Diese sehr leistungsintensiven Baugruppen sowie das nicht optimierte Consumable-Design schließen somit auch nur die Möglichkeit zum mobilen Batteriebetrieb aus. Letztendlich erkennt der Fachmann, dass sich dieses System einer mobilen Nutzung verweigert, da die Komplexität des Consumables (mehrere Fertigungsschritte, Einbringen von Hydrogelen) unzumutbare Herstellungskosten bedürfen. Der Nachweis der Amplifikation erfolgt über die Nutzung von 2 markierten Primern. Dem Fachmann ist aber bekannt, dass ein solches Nachweisverfahren auf einem Teststreifen hochproblematisch ist, da spezifische Amplifikationsprodukte nicht von den unspezifischen Amplifikaten und sog. Primer-Dimeren zu trennen sind. Damit ist ein solches Nachweissystem nicht in der Diagnostik einsetzbar.

Weiterhin gehört die Publikation von Wang und Mitarbeitern zum Stand der Technik (Wang, J. et al.; Lab on a Chip (2006)6: 46-53). Das darin vorgestellte System kann durch den Fachmann als eine Variante der zwei zuvor beschriebenen Entwicklungen identifiziert werden (z.B. gleiche Verwendung von Eis-Ventilen oder markierte Primer als Nachweissystem). Hierin werden die genauen Eigenschaften der PCR-Reaktionseinheit noch einmal konkretisiert. Es wird eine Stärke von 250 µm für die Wand zwischen Heiz-/Kühlelement und Probe angegeben. Technologieseitig wird diese Kammer und alle Kanäle durch ein CNC-Fräsverfahrens in einen Polycarbonatträger eingebracht. Damit liegt der thermische Widerstand dieser Kammer noch über dem handelsüblicher Reaktionsgefäße (Wandstärke 200 µm bis 300 µm; Material Polyethylen), in denen eine Rapid-PCR nur mittels Hochleistungsgeräten möglich ist. So schließt sich hierbei erneut die Verwendung des Konzeptes für einen Batteriebetrieb aus. Die Ineffizienz des Konzeptes wird dem Fachmann ersichtlich, wenn die Autoren auf die Schwierigkeiten beim Erreichen einer akzeptablen Kühlrate eingehen. Selbst mit aktivem Wärmetransport durch Peltierelemente und der Anbindung eines 14-Watt Lüfters (herkömmliche Lüfter für Prozessoren benötigen 6 Watt) wird in der Probe nur 2,6 K/Sekunde erreicht (siehe Rapid-PCR). Kritisch muss das Konzept auch in Hinsicht auf das formulierte Ziel der Kostenminimierung beleuchtet werden. Laut Publikation müssen unterschiedlichste Produktionsketten durchlaufen werden, um einen Chip mit diesem Konzept zu fertigen. Neben dem Fräsen der hochfeinen Kanäle müssen zusätzlich die Oberflächen poliert werden, es müssen Hydrogele eingebracht, zwei Chiphälften unter einem aufwendigen thermischen Verfahren zusammengefügt und nachträglich eine Reaktivierung der Gele realisiert werden. Darin wird dem Fachmann erkenntlich, dass dieses Chip-Konzept ein kostengünstiges Consumable unmöglich macht.

Der Stand der Technik umfasst ausserdem die Patentschrift US 6,602,473 B1, die ein batteriebetriebenes Handheld-Gerät offenbart, welches eine PCR-Reaktionskammer und Ein- und Auslassöffnungen für Primer und andere Reaktionskomponenten umfasst, wobei die Temperaturzyklen über thermoelektrische Filme/Heizelemente erzeugt werden können; sowie den anschliessenden Nachweis der Reaktionsprodukte durch Hybridisierung an spezifische Sonden auf Teststreifen ermöglicht.

### Beschreibung der Erfindung

Der Erfindung lag die Aufgabe zu Grunde, ein neuartiges mobiles gendiagnostisches Schnelltestsystems (Kombination von Hardware und Reagenzien) zu entwickeln, welches einfach zu bedienen sein soll, eine extrem schnelle diagnostische Aussage ermöglicht und sowohl in Hinblick auf das Gerät als auch in Hinblick auf den durchzuführenden Test preiswert ist. Damit soll auch die Möglichkeit gegeben sein, die Diagnostik von Infektionskrankheiten in Entwicklungsländern ohne qualitative Einschränkungen durchführen zu können.

Die Aufgabe wurde gemäß den Merkmalen der Patentansprüche gelöst. Das erfindungsgemäße mobile Schnelltestsystem für die Nukleinsäureanalytik, umfasst eine Vorrichtung zur Amplifikation und Hybridisierung von Nukleinsäuren mit Amplifikationsprimern und mindestens einer Hybridisierungssonde sowie einen Testkit zum Nachweis des Amplifikationsereignisses und ist dadurch gekennzeichnet, dass
a) das Amplifikationsprodukt und die Hybridisierungssonde mindestens jeweils eine Markierung enthalten und
b) das Testkit mindestens einen Lateral-Flow-Teststreifen umfasst, der jeweils eine Zone zur Kopplung der Markierungen enthält.

Unter dem Begriff "Markierung" ist, dass jedes Atom oder Molekül zu verstehen, das zur Erzeugung eines nachweisbaren (vorzugsweise quantifizierbaren) Signals auf einem Lateral-Flow-Teststreifen verwendet werden kann und das an eine Nukleinsäure gebunden werden kann.

Markierungen können vorzugsweise mittels Fluoreszenz, Kolorimetrie oder enzymatischer Aktivität und dergleichen nachweisbare Signale erzeugen. Bevorzugt sind Markiserungen mittels Biotin und FITC (Fluoreszeinisothiocyanat).

Die Markierung des Amplifikationsprodukts resultiert entweder durch eine Markierung eines Primers oder aus Markierung der zu bestimmenden Nukleinsäure.

Die Vorrichtung und das Testkit sind integrierte Bestandteile des mobilen Schnelltestsystems und dieses System ist ein miniaturisiertes, mobil zu betreibendes handheld-Gerät darstellt, dass während des Betriebs keine externe Spannungsquelle benötigt, sondern mittels einer Batterie oder eines Akkumulators betrieben wird.

4. Mobiles Schnelltestsystem enthält eine Reaktionskavität zur Durchführung einer Amplifikation von Nukleinsäuren, vorzugsweise mittels der Rapid-PCR-Technologie, ein oder mehrere Einlass- bzw. Auslassöffnungen für die Reaktionskavität, ein oder mehrere beheizbare Probenblöcke, die mit miniaturisierten Kühlkörpern verbunden sind und eine Möglichkeit zum Ablesen des Ergebnisses, wobei die Reaktionskavität eine Kunststoff-Folie mit einer Folienstärke, die kleiner als 300 µm, vorzugsweise kleiner als 100 µm ist, enthält. Die Kunststoff-Folie besteht vorzugsweise aus Polypropylen und wird in einer gewünschten Geometrie formstabil verschweißt und durch einen leichten Andruck von oben an den Probenblock gepresst

Ein Amplifikationsprimer ist vorzugsweise mittels Biotin markiert und die Hybridisierungssonde ist vorzugsweise mit FITC markiert und ist am 3'-Ende gegen Polymerisierung geschützt ("blockiert") ist. Diese "Blockierung" kann zum Beispiel erreicht werden durch Verwendung von nicht-komplementären Basen oder durch Anfügen einer chemischen Gruppe, wie eine Phosphatgruppe, an das 3'-Hydroxyl des letzten Nucleotids. Die Blockierung kann auch erreicht werden durch die Entfernung des 3'-OH oder durch Verwendung eines Nucleotids ohne das 3'-OH, wie ein Didesoxynucleotid.

Der Lateral-Flow-Teststreifen trägt getrennte Bindungsstellen, vorzugsweise zwei, eine Streptavidin-Stelle zur Kopplung der markierten Amplifikationsprodukte und eine Bindungsstelle zur Funktionskontrolle des Teststreifens sowie eine Zone mit konjugierten Nachweispartikeln (z.B. anti-FITC-Goldpartikel).

Gegenstand der Erfindung ist auch ein Verfahren zum Nachweis von Nukleinsäuren mittels des oben beschriebenen mobilen Schnelltestsystems mit folgenden Schritten:
- Amplifikation der Nukleinsäuren mittels der Rapid-PCR-Technologie mit Amplifikationsprimem, von denen entweder mindestens einer markiert ist oder die Nukleinsäuren markiert wurden
- Überführung eines doppelsträngigen Amplifikationsprodukts in ein einzelsträngiges DNA-Fragment (Denaturierung)
- Hybridisierung des denaturierten Amplifikationsprodukts mit mindestens einer markierten Hybridisierungssonde
- Nachweis der Nukleinsäuren auf einem Lateral-Flow-Teststreifen, der jeweils eine Zone zur Kopplung der Markierungen enthält.

Die Erfindung löst die beschriebene Problematik in idealster Weise durch die einfache und synergistische Kombination eines Gerätesystems zur Durchführung spezifischer Amplifikationsreaktionen und einer einfachen Nachweis-Chemie zur Detektion eines spezifischen Amplifikationsereignisses, wobei das Gerätesystem in Form eines sog. handheld-Systems vorliegt und damit nicht nur miniaturisiert sondern auch mobil zu betreiben ist, d.h. ohne die Notwendigkeit einer externen Spannungsquelle, sondern mittels eines Batteriebetriebes.

Die Erfindung basiert dabei auf der Nutzung der so-genannten Rapid-PCR-Technologie.

Die Erfindung löst die bestehenden Probleme eines miniaturisierten handheld-Rapid-PCR-Thermocycler in Kombination mit einem Detektionsmodul wie folgt:

Die Erfindung basiert auf einer neuartigen Anordnung und Konzeption von Heizelementen und Probenblock, welche sich für einen mobilen Einsatz in Leistung und Größe eignen, in Kombination mit einem völlig neuartigen Consumable (Reaktionskavität für die Amplifikationsreaktion) mit einem erheblich verbesserten Wärmetransfer. Dies wird erfmdungsgemäß dadurch erreicht, dass das neuartige Consumable so optimiert wird, dass das größte Problem, der physikalische Widerstand für eine effektive PCR-Temperierung, überwunden werden kann. Ein optimaler Wärmeübergang ist dann vorhanden, wenn eine Probe direkt auf den Probenblock bzw. temperierten Medium ohne störende Materialien und zusätzlichte Wärmeübergänge aufgegeben werden kann. Eine solche Realisierung verbietet sich bisher aus Gründen der Kontamination.

Eine nur minimale Störung des Wärmeübergangs wäre dann gegeben, wenn eine extrem dünne Schicht eines bioverträglichen Materials eingesetzt werden könnte. Dies wird mit der Erfindung dadurch gelöst, dass eine extrem dünnschichtige PP-Folie eingesetzt wird. Diese Folie wird dabei in eine gewünschte Geometrie gelegt bzw. gefaltet und formstabil verschweißt. Diese spezifische Geometrie zeichnet sich dadurch aus, dass sie am Boden für das einfüllbare Probenvolumen des Amplifikationsansatzes eine möglichst große flache Oberfläche besitzt. Diese große Fläche dient als Wärmeübergangsfläche zum beheizten Probenblock. Durch die ultradünne Folie wird einem Wärmeübergang ein minimaler Widerstand entgegengesetzt. Alle nicht beheizten Flächen der Reaktionskammer sind auf ein Minimum reduziert, so dass kein unnötiger Wärmeabfluss zur Umgebung stattfinden kann. So ist es möglich, innerhalb der Probe eine Heizrateneffektivität vom Probenblock auf die Probe von fast 100% zu realisieren. Vorzugsweise sind die nicht beheizte Flächen 1,4 bis 1,9mal, insbesondere 1,7mal, größer als die beheizten Flächen.

Als einzusetzenden Probenblock wird ein durch eine Batterie betriebenes Pelltierelement genutzt, welches mit einem dem Gesamtsystem angepassten Kühlkörper verschraubt ist. Die verwendete Geometrie des Probenblocks zeichnet sich als Gegenstelle des Consumables ebenfalls durch eine maximale Oberfläche aus. Das Volumen und somit auch die Kapazität des Probenblocks ist erfindungsgemäß auf ein absolutes Minimum reduziert. Der Probenblock kann damit theoretisch mit einer Heizrate von 15°C/s geheizt werden. Die Geometrie des Consumables ist erfindungsgemäß so gewählt worden, dass durch einen leichten Andruck von oben und ausgenutzte physikalische Effekte eine starke Anpressung an den Probenblock realisiert und so ebenfalls ein gleichmäßiges Heizen mit diesen Geschwindigkeiten begünstigt und realisiert wird.

Damit sind die Voraussetzungen gegeben, eine Amplifikationsreaktion mit einem kleinen mobilen Gerät unter Verwendung von einer normalen Batterie zu realisieren sowie eine ultraschnelle Reaktion durchzuführen. Dem Fachmann ist aber klar, dass allein mit einer miniaturisierten und mobilen Möglichkeit der Durchführung der PCR-Reaktion keine mobile vor-Ort- Diagnostik durchgeführt werden kann. Das System benötigt dazu auch die Detektion einer erfolgten spezifischen Targetamplifikation.

Dabei muss die Detektion integrierter Bestandteil des Gesamtsystems sein. Diese Kombination von Amplifikation und Detektion wird überraschenderweise durch die Erfindung ganz einfach mittels der Verwendung von dem Fachmann bekannten so-genannter Lateral-Flow-Teststreifen realisiert. Obwohl beide Technologien (Rapid-PCR und Lateral-Flow-Streifen) als Standard-Techniken existieren, existiert keine Verknüpfung beider Technologien in Form eines integrativen Gesamtsystems, welche die Anforderungen an eine spezifische Diagnostik erfüllen. Wie schon aufgeführt, nutzen eine Reihe ähnlicher Gerätesysteme Nachweisstrategien, die keine korrekte Diagnostik ermöglichen bzw. keine Amplifikationsreaktion von Nukleinsäuren nutzen (US 2005/0227275 A1, WO 2007/092713 A2, Chen, Z. et al.; Ann.N.Y. Acad. Sci. (March 2007)1098: 429-436, Wang, J. et al.; Lab on a Chip (2006)6: 46-53).

Eine exakte Diagnostik wird aber durch die Erfindung ermöglicht. Damit verbindet die Erfindung nicht allein ein Amplifikationsmodul mit einem Lateral Flow Streifen, sondern basiert auf einer Nachweisstrategie, die den diagnostischen Erfordernissen Rechnung trägt. Zur Unterstreichung der Unterschiede ist nachfolgend nochmals hervorgehoben, wie Lateral Flow Streifen für die DNA/RNA Diagnostik eingesetzt werden.

Die Durchführung von Gentests mittels so-genannter Lateral-Flow-Streifen wird bisher in unterschiedlicher Form propagiert. Eine Möglichkeit (Offenlegungsschrift KR1020060099022 A; Method for the detection and analysis of nucleotide sequence using membrane lateral flow, and kit for the same) nutzt Lateral-Flow-Verfahren dazu, um Nukleinsäuren zu detektieren. Dieses Verfahren bedient sich der Technologie der Hybridisierung von Nukleinsäuren an einer festen Phase. Dabei werden spezifische Fänger-Nukleinsäuren auf dem Teststreifen immobilisiert und mit einzelsträngigen Targetnukleinsäuren hybridisiert. Eine Fänger-Nukleinsäure ist eine einzelsträngige oder erst im Laufe des Verfahrens einzelsträngig werdende Ribonukleinsäure oder Desoxyribonukleinsäure, die möglicherweise chemisch modifizierte Basen oder Basenanaloga, chemisch modifizierte Zucker oder Zuckeranaloga enthält oder anderweitig modifiziert ist und sich dadurch auszeichnet, dass sie mit hoher Spezifität und Selektivität an eine bestimmte, vorgegebene Nukleinsäure oder Klasse von Nukleinsäuren bindet. Dieser Prozess erfordert dabei aber die Überführung eines doppelsträngigen DNA-Fragmentes (als Ergebnis einer spezifischen Amplifikationsreaktion) in ein einzelsträngiges DNA-Fragment. Der Prozess muss dazu im Anschluss an eine PCR-Reaktion durchgeführt werden. Eine weitere schnelle Nachweismethodik, welche sich auch dem Nachweis von Amplifikationsprodukten mittels eines Teststreifens bedient und kommerziell angeboten wird, basiert im Gegensatz zur obigen Patentschrift auf einem völlig anderen Prinzip. Hierbei erfolgt die Durchführung der PCR-Reaktion mit einem biotinylierten Primer und einem nicht-biotinylierten Primer. Nach Durchführung der PCR liegt ein PCR-Produkt vor, welches an einem Ende Biotin-markiert ist. Der Nachweis nutzt einen Teststreifen (z.B. Fa. Millenia), der zwei getrennte Bindungsstellen enthält. Eine Streptavidin-Stelle zur Kopplung des Biotin-markierten DNA Stranges und eine FITC-Bindungsstelle zur Funktionskontrolle des Teststreifens.

Der Nachweis des PCR-Produktes realisiert sich dadurch, dass man nach durchgeführter PCR den PCR-Ansatz denaturiert und mit einer zum Biotin-markierten DNA-Strang komplementären Sonde hybridisiert. Die Sonde ist FITC markiert.

Zum Nachweis wird der PCR-Ansatz mit einem Laufpuffer gemischt und auf den Teststreifen aufgetragen. Nach der Testbeschreibung bindet sich der biotinylierte DNA-Strang an die Streptavidin-Bindungsstelle des Streifens. Der Nachweis erfolgt über die FITC-Markierung der mit dem DNA-Strang hybridisierten Sonde. Es bildet sich ein typisches Signal in Form eines Streifens aus. Dieses Signal soll der spezifische Nachweis des Amplifikationsproduktes sein. Das Verfahren kombiniert aber nicht die Hybridisierung der Sonde mit dem Prozess der PCR, sondern führt dies als separaten Verfahrensschritt durch. Das Verfahren hat aber eine grundsätzliche und dramatische Fehlerquelle.

Die Detektion der nachzuweisenden Zielnukleinsäure ist nicht spezifisch. Die Ursache liegt darin, dass während der PCR entstandene Artefakte, z.B. Primer-Dimere, natürlich auch spezifisch an die Streptavidin-Bindungsstelle des Teststreifens binden und damit auch wie ein spezifisches PCR-Produkt eine positive Reaktion hervorrufen können (wie im vorangegangenem Text schon mehrfach beschrieben).

Aus diesen Darstellungen folgt, dass es zwar applikative Lösungen zur Durchführung von Gentests auf Streifen gibt, diese aber neben der Amplifikation auch noch der Denaturierung des Amplifikationsproduktes bedürfen und damit eine direkte Kombination von Amplifikation und Detektion nicht zulassen. Darüber hinaus, wie im Beispiel 2 gezeigt, sind bestimmte Verfahren nicht spezifisch und sehr problematisch in Hinblick auf die Erzeugung von falschpositiven diagnostischen Ergebnissen.

Eine neuartige und sehr elegante Möglichkeit basiert auf einer in die Amplifikationsreaktion integrierten Sondenhybridisierung zwischen Amplifikat und spezifischer Hybridisierungssonde. Der Nachweis des spezifischen Hybridisierungsereignisses erfolgt dann auf einem Teststreifen. Damit ist erstmals die Möglichkeit gegeben, ohne eine weitere Manipulation (Denaturierung doppelsträngiger DNA) und unter Umgehung der schon dargestellten Amplifikationsprobleme zu arbeiten und damit die Amplifikations/ Hybridisierungsreaktion direkt mit dem Nachweis auf einem Teststreifen kombinieren zu können. Der Nachweis eines Amplifikations-Hybridisierungsproduktes erfolgt dadurch, dass ein Amplifikationsprimer und die spezifische Hybridisierungssonde am 3'-Ende mit jeweils einem Markierungs-Molekül versehen sind (z.B. Biotin und FITC). Die Amplifikationsreaktion erfolgt unter Standardbedingungen. Dabei folgt der eigentlichen Amplifikationsreaktion ein Denaturierungsschritt zur thermischen Strangtrennung des während der PCR generierten Amplifikationsproduktes. Nach Denaturierung wird der PCR-Reaktionsansatz auf die Hybridisierungstemperatur der Sonde abgekühlt. Während dieses Schrittes bindet die Hybridisierungssonde spezifisch an den komplementären DNA-Strang des Amplifikationsproduktes. Dieser Strang trägt dabei die Biotin-Markierung, die durch den Biotin-markierten Primer in das PCR-Produkt eingebaut wurde. Nach Durchführung der Amplifikations-Hybridisierungsreaktion wird der Reaktionsansatz auf den Teststreifen übertragen. Auf dem Teststreifen kann der Nachweis des spezifischen Hybridisierungsereignisses wie folgt erfolgen: Der Teststreifen trägt z.B. in einer Ausführungsvariante zwei getrennte Bindungsstellen; eine Streptavidin-Stelle zur Kopplung der Biotin-markierten Amplifikationsprodukte und eine FITC-Bindungsstelle zur Funktionskontrolle des Teststreifens. Darüber hinaus enthält der Teststreifen eine Zone mit konjugierten Nachweispartikeln (z.B. anti-FITC-Goldpartikel). Nach dem In-Kontaktbringen des PCR-Ansatzes mit einem solchen Teststreifen können folgende Bindungsereignisse auftreten:
1. Alle FITC-markierten Nukleinsäuren (nicht hybridisierte FITC-markierte Hybridisierungssonde bzw. Hybridisierungsprodukt zwischen Biotin-markiertem DNA Strang und FITC-markierter Hybridisierungssonde) binden im unteren Probenauftragsbereich des Teststreifens an Goldpartikel, die mit anti-FITC-Antikörpern beschichtet sind.
2. Im weiteren Verlauf des Teststreifens befindet sich die Streptavidin-Bindungsstelle. An diese Bindungsstelle können sich folgende Nukleinsäuren binden:
   (a) die Biotin-markierten Primer,
   (b) die Biotin-markierten DNA-Stränge und
   (c) die Hybridisierungsprodukte zwischen Biotin-markiertem DNA Strang und FITC-markierter Hybridisierungssonde.

   Ein Nachweissignal kann aber nur dann sichtbar werden, wenn das spezifische
   Hybridisierungsprodukt zwischen Biotin-markiertem DNA Strang und FITC-markierter Hybridisierungssonde vorliegt, da nur dieses Produkt auch mit dem Nachweissystem (FITC- anti-FITC- Goldpartikel) gekoppelt ist.
3. Im weiteren Verlauf des Teststreifens binden dann noch überschüssige mit anti-FITC-Antikörpern beschichte Goldpartikel, welche als Kontrolle zur Funktionsfähigkeit des Teststreifens dienen.

Diese Ausführungsvariante ist, wie schon erwähnt, sehr elegant und birgt nicht die potenzielle Gefahr von falsch-positiven Signalen. Sie ist damit für das erfindungsgemäße mobile Nachweissystem die zu favoritisierende Variante der Nutzung eines Lateral-Flow-Teststreifens.

Die Erfindung ist als Kombination des neuartigen Verfahrens zur Detektion von Amplifikaten und einer neuartigen Vorrichtung zur mobilen Anwendung zu verstehen. In der erfindungsgemäßen Umsetzung der Vorrichtung werden die Detektionsstrategie mittels Lateral Flow Streifen, eine intelligente Probenaufgabe und eine energieeffiziente Temperierung von Probenvolumina in einem kostengünstigen -weil einfach herzustellenden- Consumable vereinigt.

Eine erfindungsgemäße Ausführung der Vorrichtung sieht wie folgt aus:

Das Consumable stellt eine konstruktive Integration der Funktionsmodule für die Aufgabe des inital hergestellten Reaktionsgemisches (zu untersuchende Nukleinsäure, dNTP's, Primer, Hybridisierungssonde sowie Amplifikationspuffer), für die Durchführung einer energetisch effizienten Amplifikationsreaktion, für die Aufbewahrung und die Applikation eines Laufpuffers sowie für die Nachweisreaktion mittels Teststreifen dar. Das batteriebetriebene Gerät ist so ausgeführt, dass es ein ideales Mittel zur Prozessierung der Funktionsabschnitte des Consumables darstellt. Der Ablauf der Prozessierung beginnt mit Aufgabe des Reaktionsgemisches über die Einlassöffnung, wobei das Gemisch unmittelbar oder über weiten Prozessstrecken in die Amplifikationskammer geleitet wird. Nach erfolgter Amplifikations-/ Hybridisierungsreaktion wird der Reaktionsansatz über die Auslassöffnung auf den Teststreifen überführt. Aus einem separaten Reservoir wird dann nachfolgend der Laufpuffer ebenfalls auf den Testreifen zugeführt, wobei in einer Ausführungsvariante der Laufpuffer zuvor die Reaktionskammer durchlaufen kann. Das Reservoir für den Laufpuffer befindet sich ebenfalls in der Kartusche. Nach Beendigung der Testreifen-Reaktion wird das diagnostische Ergebnis von einer Sichtzone abgelesen. Die Reaktionskartusche, welche das Amplifikationsmodul und den Teststreifen sowie das Reservoir für den Laufpuffer enthält, ist vorzugsweise ein Einwegartikel, das heißt, dass für jede Reaktion eine neue in das Basis- Gerät eingelegt und nach Testlauf verworfen wird.

Die erfindungsgemäße Vorrichtung ist auch ohne das erfindungsgemäße Nachweissystem Gegenstand der beschriebenen Erfindung und kann auch mit dem oben genannten Nachweissystem der Fänger-Nukleinsäuren kombiniert werden. Damit wird ein System bereitgestellt, dass aus der erfindungsgemäßen Vorrichtung gemäß einem der Ansprüche 3 bis 12 und einem Teststreifen, auf dem spezielle Fängernukleinsäuren immobilisiert sind, besteht und mit dem der Nachweis der zu bestimmenden Nukleinsäuren durch die Hybridisierung mit den Fängernukleinsäuren auf dem Teststreifen erfolgt.

Nachfolgend werden diese erfindungsgemäßen Ausführungsvarianten erläutert, ohne die Erfindung auf diese Varianten zu beschränken.

### Ausführungsbeispiele

### Beispiel 1

In die Einlassöffnung erfolgt die Zuführung des Reaktionsgemisches. Die Einlassöffnung wird durch einen in das Gerät integrierten Glühdraht verschweißt. Der Glühdraht wird dabei durch einen einfachen Druckmechanismus zur Einlassöffnung bewegt. In dieser Ausführung ist sowohl das für die Amplifikations-/ Hybridisierungsreaktion erfindungsgemäß eingesetzte Consumable (Reaktionskavität), als auch der Teststreifen hintereinander in eine Reaktionskartusche verbracht.

### Beispiel 2

Die Ausführungsvariante des Consumables ist so gestaltet, dass eine oder alle Flüssigkeiten auf dem Consumable über neuartige Fluidikstrukturen von einem Funktionsmodul zum nächsten transportiert werden. Diese Strukturen werden fertigungstechnisch dadurch erzeugt, dass zwei Oberflächen aufeinander gebracht werden, wobei mindestens eine dieser Flächen aus einem plastisch oder elastisch deformierbaren Material besteht. An der Begrenzung der Strukturen, zum Beispiel der Kanal- oder Kammerränder, werden die beiden Oberflächen kraft- und/oder formschlüssig miteinander verbunden. Wird der feine Spalt, den diese Struktur darstellen kann, mit fluidischem Druck beaufschlagt, so wölbt sich mindestens eine der Oberflächen auf und gibt einen größeren Spalt zum Durchfluss zwischen beiden Flächen frei. Durch dieses neuartige Konzept wird eine kostengünstige Fertigung von Fluidikelementen auf dem Consumable möglich.

### Beispiel 3

In einer weiteren Ausführungsvariante stellt die Reaktionskammer einen Durchbruch oder eine Vertiefung im Trägermaterial des Consumables dar. Ein Verschluss der Kammer wird erreicht, indem die darüber liegende Folie auf die Ränder des Durchbruchs, respektive Vertiefung, angedrückt wird. In einer Ausführung dieser Variante wird der Druck durch den Probenblock erzeugt, so dieser sowohl den Wärmeübertrag als auch den Kammerverschluss bewerkstelligt. Die erhöhten Anforderungen an die Druckdichtigkeit während der PCR wird erreicht, indem der Probenblock konvex ausgeführt und die Kammeröffnung kreisrund ist.

### Beispiel 4

Die Aufbewahrung und die Applikation des Laufpuffers kann in einer Ausführungsvariante folgendermaßen ausgestaltet sein: zwischen der Folie und dem Trägermaterial wird durch raumfordernde Herstellungsverfahren ein Hohlraum geschaffen. Dieser wird bei der Herstellung/ Bestückung des Consumables mit Laufpuffer befüllt. Die Auslassöffnung des Reservoirs ist mit einem druckabhängigen Ventil verschlossen, dass z.B. durch eine wiederlösbare Schweißnaht umgesetzt sein kann. Für eine Zuführung des Puffers in die Reaktionskammer wird die Puffcrkammcr durch das Gerät oder durch den Anwender derart mit mechanischem Druck beaufschlagt, dass sich das Ventil öffnet und der Puffer zum Teststreifen bewegt wird.

### Beispiel 5

In dieser Ausführungsvariante sind bewegliche Kolben und dazugehörige hohle Zylinder Teil des Consumables. Dabei können fertigungsseitig diese Zylinder mit Flüssigkeiten gefüllt sein (z.B. Laufpuffer, Reaktanten). Die Einlassöffnung des Consumables ist derart geformt, dass ein Probenaufgabewerkzeug formschlüssig angeschlossen werden kann. Dieses Werkzeug ist ebenso aus einem Hohlzylinder mit Kolben zusammengesetzt. Für eine Probenentnahme wird der Kolben in dem Zylinder aufgezogen und dann an das Consumable angeschlossen. Die Kolben des Consumables und des Entnahmewerkzeuges können durch den Nutzer und oder durch das Gerät bewegt werden. Durch die Kolbenbewegung am Probenaufgabewerkzeug wird die Probe in die Reaktionskammer gedrückt. Luft die so aus der Kammer bzw. aus den Fluidikbereichen verdrängt werden muss, entweicht in luftfreie Räume und/ oder aus Auslassöffnungen die fluidisch hinter dem Nachweisbereich gelegen sind. Als dann erfolgt das Aufpressen eines oder mehrerer Probenblöcke von einer oder mehreren Seiten der Reaktionskammer. Mit diesem Prozess wird wie in Beispiel 4 die Reaktionskammer druckdicht verschlossen, so dass eine energetisch günstige Temperierung der Probe erfolgen kann. Mit dem Entfernen der Probenblöcke kann nun die Probe weiter Richtung Nachweiskammer gedrückt werden. Dazu kann der Kolben des Probenaufgabewerkzeuges und/ oder der Kolben des Zylinders mit dem Laufpuffer bewegt werden. Luft und oder Flüssigkeiten aus den beschriebenen Zylindern verdrängt die Probe aus der Reaktionskammer und bewegt sie auf den Teststreifen. Weiterer Laufpuffer aus dem entsprechenden Zylinder wird durch die Bewegung der Kolben auf den Teststreifen gebracht. Über ein Sichtfenster im Nachweisbereich kann der Anwender das Ergebnis der Detektion ablesen. Wie das Aufgabewerkzeug, kann in einer weiteren Ausführungsvariante auch der Nachweisbereich von dem Gesamtconsumable reversibel abgelöst bzw. angeschlossen und durch ein alternatives Nachweissystem ersetzt werden.

Mit der erfindungsgemäßen Kombination von rapid-PCR und Detektion des Amplifikationsereignisses mittels eines mobilen und batteriebetriebenen handheld-Systems ist erstmals die Voraussetzung für eine ganz einfache und preiswerte gendiagnostische Vor-Ort-Analyse gegeben. Dabei ist das handheld-Gerät natürlich sehr viel preiswerter als die bisher vorgeschlagenen hochaufgerüsteien Gerätesysteme, insbesondere für militärische Anwendungen. Dies soll auch die Nutzung von gendiagnostischen Schnelltests in Entwicklungsländern universell ermöglichen. Durch das erfindungsgemäße Verfahren wird die Durchführung einer extrem schnellen Amplifikationsreaktion ermöglicht. Die nachfolgende Detektion auf einem Testsreifen ist ebenfalls sehr schnell und robust. Damit stellt die Erfindung ein wirkliches Schnelltestsystem dar.

### Erläuterung der Figuren:

Die Erfindung wird anhand von zwei Figuren näher erläutert. Im Anhang sind die Prinzipskizzen für das erfindungsgemäße Mittel dargestellt. Die Zeichnungen gelten dabei nicht als Limitierung für andere Ausführungsformen. Figur 1 zeigt die schematische Darstellung der Kartusche. Figur 2 zeigt die schematische Darstellung des Gerätes (Spannungsquelle und Reaktionskartusche).

### Bezugszeichenliste:

A: Einlassöffnung
B. Consumable für die Amplifikations- Hybridisierungsreaktion
C: Auslassöffnung und Verbindung zwischen Consumable und Teststreifen
D: Reservoir für Laufpuffer
E: Auslassöffnung vom Reservoir für Laufpuffer mit Zuführung zum Testsreifen
F: Teststreifen
G: Sichtfenster für die Detektion des Nachweissignals auf dem Teststreifen
H: Batterie als Spannungsquelle
I: Heiz/ Kühlelement

## Patentansprüche

1. Mobiles Schnelltestsystem für die Nukleinsäureanalytik in Form eines mobil zu betreibenden Handheld-Geräts, das während des Betriebs keine externe Spannungsquelle benötigt, umfassend eine Vorrichtung zur Amplifikation und Hybridisierung von Nukleinsäuren sowie einen Testkit zum Nachweis des Amplifikationsereignisses, wobei die Vorrichtung eine Reaktionskavität zur Durchführung einer Amplifikation von Nukleinsäuren mittels der Rapid-PCR-Technologie, ein oder mehrere Einlass- bzw. Auslassöffnungen für die Reaktionskavität, ein oder mehrere beheizbare Probenblöcke, die mit miniaturisierten Kühlkörpern verbunden sind und eine Möglichkeit zum Ablesen des Ergebnisses umfasst, **dadurch gekennzeichnet, dass**
a) die beheizbaren Probenblöcke konvex ausgeführt sind und die nicht beheizten Flächen 1,4 bis 1,9 mal größer als die beheizten Flächen sind und
b) die Reaktionskavität eine Kunststoff-Folie mit einer Folienstärke, die kleiner als 300 µm ist, enthält und
c) der Nachweis des Amplifikationsereignisses mittels eines Lateral-Flow-Teststreifens erfolgt.

2. Mobiles Schnelltestsystem nach Anspruch 1, **dadurch gekennzeichnet, dass** bei den beheizbaren Probenblöcken die nicht beheizten Flächen 1,7 mal größer als die beheizten Flächen sind.

3. Mobiles Schnelltestsystem nach Anspruch 1, **dadurch gekennzeichnet, dass** die Reaktionskavität eine Kunststoff-Folie mit einer Folienstärke, die kleiner als 100 µm ist, enthält.

4. Mobiles Schnelltestsystem nach einem der Ansprüche 1 bis 3, **dadurch gekennzeichnet, dass** die Vorrichtung und das Testkit integrierte Bestandteile des mobilen Schnelltestsystems sind und dieses System mittels einer Batterie oder eines Akkumulators betrieben wird.

5. Mobiles Schnelltestsystem nach einem der Ansprüche 1 bis 4, **dadurch gekennzeichnet, dass** der Lateral-Flow-Teststreifen jeweils eine Zone zur Kopplung von Markierungen enthält.

6. Mobiles Schnelltestsystem nach Anspruch 1 oder 3, **dadurch gekennzeichnet, dass** die Kunststoff-Folie aus Polypropylen besteht und in einer gewünschten Geometrie formstabil verschweißt und durch einen leichten Andruck von oben an den Probenblock gepresst wird.

7. Mobiles Schnelltestsystem nach einem der Ansprüche 1 bis 6, **dadurch gekennzeichnet, dass** die Reaktionskavität und der Teststreifen in einer Reaktionskartusche angeordnet sind und dass die Kunststoff Folie mit der Reaktionskartusche Verbindungskanäle erzeugt.

8. Mobiles Schnelltestsystem nach einem der Ansprüche 1 bis 7, **dadurch gekennzeichnet, dass** es zusätzlich ein Reservoir und eine Auslassöffnung für einen Laufpuffer umfasst.

9. Mobiles Schnelltestsystem nach Anspruch 8, **dadurch gekennzeichnet, dass** es verschließbare Verbindungskanäle zwischen Reaktionskavität, Teststreifen und Laufpuffer-Reservoir enthält.

10. Mobiles Schnelltestsystem nach einem der Ansprüche 1 bis 9, **dadurch gekennzeichnet, dass** die Reaktionskavität zwei Oberflächen enthält, die an Kanal- oder Kammerrändern kraft- und/oder formschlüssig miteinander verbunden werden können, wobei mindestens eine dieser Flächen aus einem plastisch oder elastisch deformierbaren Material besteht.

11. Mobiles Schnelltestsystem nach einem der Ansprüche 1 bis 10, **dadurch gekennzeichnet, dass** die Reaktionskavität eine Vertiefung enthält.

12. Mobiles Schnelltestsystem nach einem der Ansprüche 1 bis 10, **dadurch gekennzeichnet, dass** die Reaktionskavität bewegliche Kolben und dazugehörige hohle Zylinder zur Aufbewahrung von Reaktanten enthält.

13. Mobiles Schnelltestsystem nach einem der Ansprüche 1 bis 12, **dadurch gekennzeichnet, dass** der beheizbare Probenblock, der mit einem miniaturisierten Kühlköper verbunden ist, ein batteriebetriebenes Peltierelement darstellt, mit dem eine Heizrate von bis zu 15°C/s möglich ist.

14. Mobiles Schnelltestsystem nach einem der Ansprüche 1 bis 13, **dadurch gekennzeichnet, dass** der Lateral-Flow-Teststreifen zwei getrennte Bindungsstellen trägt, eine Streptavidin-Stelle zur Kopplung der markierten Amplifikationsprodukte und eine Bindungsstelle zur Funktionskontrolle des Teststreifens sowie eine Zone mit konjugierten Nachweispartikeln, vorzugsweise anti-FITC-Goldpartikel.

## Claims

1. A mobile quick test system for nucleic acid analysis in the form of a handheld equipment to be operated in a mobile manner, which does not require an external voltage source during operation, comprising a device for amplification and hybridization of nucleic acids as well as a test kit for proof of the amplification event, the device comprising a reaction well for performance of an amplification of nucleic acids by means of the rapid PCR technology, one or more inlet and/or outlet openings for the reaction well, one or more heatable specimen blocks connected with miniaturized cooling elements, and a possibility for reading out the result, **characterized in that**
a) the heatable specimen blocks are formed convexly, and the unheated surfaces are 1.4 up to 1.9 times greater than the heated surfaces, and
b) the reaction well comprises a plastic foil with a foil thickness of less than 300 µm, and
c) the proof of the amplification event occurs by means of a lateral flow strip.

2. The mobile quick test system according to claim 1, **characterized in that** on the heatable specimen blocks the unheated surfaces are 1.7 times greater than the heated surfaces.

3. The mobile quick test system according to claim 1, **characterized in that** the reaction well comprises a plastic foil with a foil thickness of less than 100 µm.

4. The mobile quick test system according to any one of claims 1 to 3, **characterized in that** the device and the test kit are integrated component parts of the mobile quick test system, and said system is operated by means of a battery or an accumulator.

5. The mobile quick test system according to any one of claims 1 to 4, **characterized in that** the lateral flow strip each comprises one zone for coupling of markings.

6. The mobile quick test system according to claim 1 or 3, **characterized in that** the plastic foil is comprised of polypropylene, and in a desired geometry is welded in a dimensionally stable manner, and pressed to the specimen block by a slight pressure from above.

7. The mobile quick test system according to any one of claims 1 to 6, **characterized in that** the reaction well and the lateral flow strip are defined in a reaction cartridge, and that the plastic foil with the reaction cartridge produces communication passages.

8. The mobile quick test system according to any one of claims 1 to 7, **characterized in that** in addition it comprises a reservoir and an outlet opening for a running buffer.

9. The mobile quick test system according to claim 8, **characterized in that** it comprises lockable communication passages between reaction well, lateral flow strip and running buffer reservoir.

10. The mobile quick test system according to any one of claims 1 to 9, **characterized in that** the reaction well comprises two surfaces, which can be connected with each other in a positive and/or non-positive manner on passage or chamber borders, at least one of said surfaces comprising a plastic or elastically deformable material.

11. The mobile quick test system according to any one of claims 1 to 10, **characterized in that** the reaction well comprising a recess.

12. The mobile quick test system according to any one of claims 1 to 10, **characterized in that** the reaction well comprises movable pistons and associated hollow cylinders for the storage of reactants.

13. The mobile quick test system according to any one of claims 1 to 12, **characterized in that** the heatable specimen block, which is connected with a miniaturized cooling element, constitutes a battery-operated peltier element with which a heating rate of up to 15°C/s is possible.

14. The mobile quick test system according to any one of claims 1 to 13, **characterized in that** the lateral flow strip carries two separate binding sites, a streptavidin site for coupling of the marked amplification products, and a binding site for functional control of the lateral flow strip, as well as a zone with conjugated tracing particles, preferably anti-FITC gold particles.

## Revendications

1. Système mobile de test rapide pour l'analyse de l'acide nucléique sous forme d'un dispositif portatif à exploiter d'une manière mobile ne nécessitant pas une source externe de tension lors du fonctionnement, comportant un dispositif pour l'amplification et la hybridation des acides nucléiques ainsi qu'un kit d'essai pour la preuve de l'événement de l'amplification, le dispositif comportant un puits de réaction pour exécution d'une amplification des acides nucléiques au moyen de la technologie PCR rapide, une ou plusieurs ouvertures d'entrée et/ou de sortie pour le puits de réaction, un ou plusieurs blocs d'échantillons pouvant être chauffés, qui sont reliés avec des éléments de refroidissement miniaturisés, et une possibilité de lecture des résultats, **caractérisé en ce que**
a) les blocs d'échantillon pouvant être chauffés sont formés d'une manière convexe et les surfaces non chauffées sont 1.4 à 1.9 fois plus grandes que les surfaces chauffées, et
b) le puits de réaction comprend un film plastique avec une épaisseur de film inférieure à 300 µm, et
c) la preuve de l'événement de l'amplification a lieu au moyen d'un flux latéral et bande fondée.

2. Système mobile de test rapide selon la revendication 1, **caractérisé en ce que** sur les blocs d'échantillons pouvant être chauffés, les surfaces non chauffées sont 1.7 fois plus grandes que les surfaces chauffées.

3. Système mobile de test rapide selon la revendication 1, **caractérisé en ce que** le puits de réaction comprend un film plastique avec une épaisseur de film inférieure à 100 µm.

4. Système mobile de test rapide selon l'une quelconque des revendications 1 à 3, **caractérisé en ce que** le dispositif et le kit d'essai sont des composants intégrés du système mobile de test rapide, et ledit système est exploité au moyen d'une batterie ou une accumulateur.

5. Système mobile de test rapide selon l'une quelconque des revendications 1 à 4, **caractérisé en ce que** le flux latéral et bande fondée chacun comprend une zone pour l'accouplement des marquages.

6. Système mobile de test rapide selon la revendication 1 ou 3, **caractérisé en ce que** le film plastique est constitué de polypropylène, et dans une géométrie souhaitée est soudé dans une forme stable, et pressé au bloc d'échantillon par une pression légère d'en haut.

7. Système mobile de test rapide selon l'une quelconque des revendications 1 à 6, **caractérisé en ce que** le puits de réaction et le flux latéral et bande fondée sont disposés dans une cartouche de réaction, et que le film plastique génère des passages de communication avec la cartouche de réaction.

8. Système mobile de test rapide selon l'une quelconque des revendications 1 à 7, **caractérisé en ce qu'**il comprend en outre un réservoir et une ouverture de sortie pour un tampon de migration.

9. Système mobile de test rapide selon la revendication 8, **caractérisé en ce qu'**il comprend des passages de communication verrouillables entre le puits de réaction, le flux latéral et bande fondée et le réservoir de tampon de migration.

10. Système mobile de test rapide selon l'une quelconque des revendications 1 à 9, **caractérisé en ce que** le puits de réaction comprend deux surfaces qui peuvent être reliées l'une avec l'autre à engagement positif et/ou par adhérence aux bords de passage ou de chambre, au moins une desdites surfaces comportant une matière plastique ou déformable élastiquement.

11. Système mobile de test rapide selon l'une quelconque des revendications 1 à 10, **caractérisé en ce que** le puits de réaction comprend une rainure.

12. Système mobile de test rapide selon l'une quelconque des revendications 1 à 10, **caractérisé en ce que** le puits de réaction comprend des pistons mobiles et des cylindres creux associés pour le stockage des réactants.

13. Système mobile de test rapide selon l'une quelconque des revendications 1 à 12, **caractérisé en ce que** le bloc d'échantillon pouvant être chauffé, qui est relié à un élément de refroidissement miniaturisé, comprend un élément Peltier alimenté par batterie avec lequel un taux de chauffage jusqu'à 15°C/s est possible.

14. Système mobile de test rapide selon l'une quelconque des revendications 1 à 13, **caractérisé en ce que** le flux latéral et bande fondée porte deux sites de liaison séparés, un site de Streptavidine pour accouplement des produits marqués d'amplification, et un site de liaison pour contrôle de fonctionnement du flux latéral et bande fondée ainsi qu'une zone avec particules de détection conjuguées, de préférence des particules d'or anti-FITC.
